# EUROPÄISCHE PATENTSCHRIFT

(11) **EP 3 566 552 B1**
(45) Veröffentlichungstag und Bekanntmachung des Hinweises auf die Patenterteilung: **24.03.2021**
(21) Anmeldenummer: 17832909.0
(22) Anmeldetag: 20.12.2017
(51) Int. Cl.: H05H 1/24, A61N 1/44, A61L 2/14

(54) **FLÄCHIGES FLEXIBLES AUFLAGESTÜCK FÜR EINE DIELEKTRISCH BEHINDERTE PLASMABEHANDLUNG**
FLAT FLEXIBLE SUPPORT PIECE FOR A DIELECTRICALLY IMPEDED PLASMA TREATMENT
ÉLÉMENT D'APPUI SOUPLE PLAT POUR UN TRAITEMENT PAR PLASMA À BARRIÈRE DIÉLECTRIQUE

(30) Priorität: 05.01.2017 DE 102017100161
(43) Veröffentlichungstag der Anmeldung: 13.11.2019
(73) Patentinhaber: Cinogy GmbH, 37115 Duderstadt (DE)
(72) Erfinder: HAHNL, Mirko, 37339 Berlingerode (DE); STORCK, Karl-Otto, 37115 Duderstadt (DE); TRUTWIG, Leonhard, 37115 Duderstadt (DE); WANDKE, Dirk, 37308 Heilbad Heiligenstadt (DE)
(74) Vertreter: Gramm, Lins & Partner Patent- und Rechtsanwälte PartGmbB
(86) Internationale Anmeldenummer: PCT/DE2017/101097
(87) Internationale Veröffentlichungsnummer: WO 2018/127257

(56) Entgegenhaltungen:
- EP-A1- 3 051 926
- WO-A2-2016/186501
- DE-A1- 10 136 403
- DE-A1-102011 105 713
- DE-A1-102014 013 716
- US-A1- 2015 157 870
- US-A1- 2016 331 989

## Beschreibung

Die Erfindung betrifft ein flächiges flexibles Auflagestück mit einer mit einer Hochspannung versorgbaren Elektrodenanordnung für eine dielektrisch behinderte Plasmabehandlung einer zu behandelnden Oberfläche, wobei die Elektrodenanordnung wenigstens eine flächige Elektrode und eine Anlagefläche für die zu behandelnde Oberfläche aufweisende Dielektrikumsschicht aus einem flächigen flexiblen Material aufweist, die die wenigstens eine Elektrode elektrisch von der zu behandelnden Oberfläche so abschirmt, dass nur ein dielektrisch behinderter Stromfluss zwischen der wenigstens einen Elektrode und der zu behandelnden Oberfläche möglich ist, wenn in einem Gasraum zwischen der Elektrodenanordnung und der zu behandelnden Oberfläche ein Plasmafeld durch die Hochspannung der Elektrode entsteht.

Die Behandlung von Oberflächen, zu denen auch die menschliche Haut gehört, mit einem dielektrisch behinderten Plasmafeld ist bekannt. Beispielsweise offenbart DE 10 2009 060 627 B4 ein flächiges flexibles Auflagestück mit den obigen Merkmalen. Die dabei vorzugsweise vom Dielektrikum vollständig umschlossene flächige und flexible Elektrode wird in geeigneter Weise über ein Hochspannungskabel mit einer Hochspannung versorgt, die für die Ausbildung des dielektrisch behinderten Plasmafelds benötigt wird. Die Kontaktierung der Elektrode kann an einem in einem Gehäuse angebrachten Elektrodenanschluss erfolgen, der aus der Dielektrikumsschicht herausragt. In einer durch EP 2 723 447 B1 bekannten Anordnung erfolgt die Kontaktierung der Elektrode mittels eines Schneidkontakts, der durch die Dielektrikumsschicht hindurchgedrückt wird, um so durch das Dielektrikum hindurch die Elektrode zu kontaktieren. Dieser Schneidkontakt ist in einem Kontaktgehäuse angeordnet, durch den eine Kontaktierung einer Bedienperson mit der Hochspannung sicher unterbunden wird.

EP 2 946 641 B1 offenbart für eine speziell mit einem Dielektrikum in Kugelform beschriebenen Elektrodenanordnung, dass die Hochspannung im Griff eines Gehäuses generiert wird, um dann mittels einer im Gehäuse geführten Leitung auf die Elektrode geleitet zu werden. Mit dem Griff des Gehäuses kann die kugelförmige Elektrodenanordnung über die zu behandelnde Oberfläche, beispielsweise eine Hautfläche, flächendeckend bewegt werden.

US 2016/0331989 A1 offenbart eine Anordnung für die Behandlung mit einem dielektrisch behinderten Plasma, bei der ein flexibles Auflagestück mit einer in ein Dielektrikum eingebetteten Elektrode flächig ausgebildet ist und mittels einer umlaufenden Klebeschicht auf der Haut eines Patienten befestigt werden kann. Die Ansteuerung der Elektrode in dem Auflagestück erfolgt über ein externes Steuergerät. In einer Ausführungsform befindet sich das Steuergerät zur Erzeugung der Hochspannungssignale für die Elektrode zusammen mit einer flachen Batterie in einer Tasche, die mit dem Auflagestück über eine Steckverbindung verbindbar ist. Über die Steckverbindung werden daher Hochspannungssignale auf die in dem Auflagestück befindliche Elektrode übertragen.

Der vorliegenden Erfindung liegt die Aufgabe zugrunde, die Zuführung der Hochspannung zu einer Elektrode der Elektrodenanordnung einfacher und sicherer zu gestalten.

Zur Lösung dieser Aufgabe ist erfindungsgemäß ein flächiges, flexibles Auflagestück der eingangs erwähnten Art dadurch gekennzeichnet, dass das Auflagestück eine Hochspannungsstufe zur Erzeugung einer Hochspannung aufweist, deren Ausgang mit der wenigstens einen Elektrode durch ein als leitender Abschnitt innerhalb der Dielektrikumsschicht ausgebildetes Verbindungsstück auf dem Auflagestück verbunden ist.

Das erfindungsgemäße Auflagestück beinhaltet somit nicht nur die Elektrodenanordnung, sondern auch die Hochspannungsstufe. Dies hat den Vorteil, dass der Ausgang der Hochspannungsstufe auf kürzestem Wege mit der wenigstens einen Elektrode der Elektrodenanordnung verbunden werden kann. Dies kann über ein entsprechend isoliertes Leitungsstück erfolgen, das sich als leitender Abschnitt innerhalb der Dielektrikumsschicht befindet. Demgemäß ist das die Hochspannung führende Verbindungsstück von dem die wenigstens eine Elektrode umgebenden und isolierenden Dielektrikum umgeben und damit sicher gegen Berührung isoliert. Bei dem erfindungsgemäßen Auflagestück stellt sich somit das Problem der Hochspannungszuführung über eine längere Strecke nicht. Der kurze leitende Abschnitt zwischen der Hochspannungsstufe und der wenigstens einen Elektrode der Elektrodenanordnung kann eine eigene Isolierung aufweisen, ist jedoch vorzugsweise mit dem Dielektrikum umgeben, das auch die wenigstens eine Elektrode umgibt. Hierzu kann das Verbindungsstück eine in die Dielektrikumsschicht eingebrachte Leiterbahn sein. Die Leiterbahn kann als vorgefertigtes Bauelement zur Verbindung der Hochspannungsstufe und der wenigstens einen Elektrode angeordnet und dann von dem Dielektrikum, vorzugsweise im Spritzgussverfahren, umgeben werden. Es ist aber auch möglich, den das Verbindungsstück ausmachenden leitenden Abschnitt innerhalb der Dielektrikumsschicht aus einem spritzgegossenen Kunststoffmaterial mit leitenden Zusätzen auszubilden. In diesem Fall ist es zweckmäßig, ein dreistufiges Spritzgießen durchzuführen, mit dem eine untere Lage der Dielektrikumsschicht, dann die leitende Schicht des Verbindungsstücks und anschließend die obere Lage der Dielektrikumsschicht gespritzt wird. Mit der Herstellung des leitenden Abschnitts kann zugleich die wenigstens eine Elektrode ebenfalls aus einem Kunststoffmaterial mit leitenden Zusätzen und vorzugsweise in demselben Spritzgussschritt wie das Verbindungsstück hergestellt werden.

In einer Ausführungsform der Erfindung kann das flächige flexible Auflagestück ohne Anschlüsse nach außen ausgebildet werden, wenn es ferner Batterien für eine Gleichspannungsversorgung und eine Steuerschaltung zur Umwandlung der Gleichspannung in Wechselspannungssignale einer höheren Spitzenspannung aufweist. Die so gebildeten Wechselspannungssignale können dann auf die Hochspannungsstufe gelangen. Die Batterien können ebenfalls in das Dielektrikum eingebettet werden, sodass die Verbindungsleitungen zwischen den Batterien und der Steuerschaltung sowie zwischen der Steuerschaltung und der Hochspannungsstufe in der gleichen Weise wie der leitende Abschnitt des Verbindungsstücks hergestellt werden können. Die Batterien und Teile der Steuerschaltung, insbesondere ein Mikroprozessorchip, können in geeigneter Weise in das Dielektrikum eingesetzt werden, insbesondere wenn das Dielektrikum im Spritzgussverfahren ausgebildet wird. In der Ausführungsform mit eingesetzten Batterien ist das flächige flexible Auflagestück von jeglicher Spannungsversorgung unabhängig. Die sehr preisgünstig herzustellenden Batterien, Steuerschaltung und Hochspannungsstufe ermöglichen es, das flächige flexible Auflagestück als Einmalartikel auszubilden, was insbesondere bei der Verwendung als Wundauflage vorteilhaft ist, weil jeglicher Wiederaufbereitungsaufwand entfallen kann. Gegebenenfalls kann vorgesehen werden, die Batterien aus dem Material des Dielektrikums entnehmbar zu gestalten, um die Batterien getrennt entsorgen bzw. recyceln zu können. Die Batterien können übliche Einmalbatterien, aber auch aufladbar (Akkumulatoren) sein.

In einer Zwischenstufe weist das flächige flexible Auflagestück lediglich Anschlüsse für eine Wechsel-Versorgungsspannung auf, aus der die Hochspannungsstufe dann die benötigte Hochspannung generiert. In diesem Fall entfällt die Entsorgung der Batterien und der Anschluss der Wechsel-Versorgungsspannung kann in üblicher Technik gestaltet werden, weil die Handhabung einer Hochspannung entfällt.

In einer Ausführungsform der Erfindung gelangt an den Eingang der Hochspannungsstufe eine Wechselspannung. Die Hochspannungsstufe kann hieraus Wechselspannungsimpulse generieren, die eine Frequenz zwischen 100 Hz und 100 MHz aufweisen und vorzugsweise als schmale Nadelimpulse mit schnell abklingenden Wechselspannungsschwingungen ausgebildet sind. Die verwendeten Hochspannungen liegen zweckmäßig zwischen 1 kV und 100 kV.

In einer Ausführungsform der Erfindung weist die Elektrodenanordnung wenigstens zwei Elektroden auf, die phasenverschoben mit der Wechsel-Hochspannung versorgbar sind. Wenn Wechselspannungsimpulse verwendet werden, kann es zweckmäßig sein, die Wechselspannungsimpulse gegenphasig den wenigstens zwei Elektroden zuzuleiten, sodass sich zwischen den Elektroden eine verdoppelte Spannung ausbildet. Auf diese Weise lässt sich die Effizienz für die Plasmabildung gegenüber der zu behandelnden Oberfläche, insbesondere der Hautoberfläche, verbessern, auch wenn die zu behandelnde Oberfläche als Gegenelektrode dient, die etwa auf Massepotential liegt. Das Massepotential ist bei genau gegenphasigen Impulsen ein Nullpotential, das mittig zwischen den beiden Spitzenspannungen der gegenphasigen Impulse liegt. Dieses Mittenpotential stellt sich auch dann ein, wenn die Oberfläche, also beispielsweise der zu behandelnde menschliche oder tierische Körper, nicht gesondert auf Massepotential/Erdpotential gelegt wird.

Das flächige flexible Auflagestück gemäß der vorliegenden Erfindung kann in einer Ausführungsform als eine Wundauflage mit einem wundverträglichen Material gebildet sein. Das wundverträgliche Material kann dabei das Material der Dielektrikumsschicht sein. Es ist aber auch möglich, auf die Auflagefläche des Dielektrikums, die zur Auflage auf der zu behandelnden Oberfläche vorgesehen ist, ein wundverträgliches Material aufzubringen.

In einer einfachen und bevorzugten Ausführungsform der Erfindung ist das Dielektrikum als Spritzgussteil ausgebildet und umschließt sowohl die wenigstens eine Elektrode als auch das Hochspannungsteil allseitig. Soweit auch ein Steuerteil und ggf. Batterien vorgesehen sind, können auch diese von dem Dielektrikum umschlossen sein, sodass das Dielektrikum als Verkapselung für alle elektrischen Teile des Auflagestücks fungiert.

In den beigefügten Zeichnungen sind mehrere Ausführungsformen zur Erläuterung der Erfindung beschrieben. Es zeigen:
- Figur 1: eine erste Ausführungsform eines flächigen flexiblen Auflagestücks in einer Draufsicht und mehreren Schnittdarstellungen,
- Figur 2: eine zweite Ausführungsform des flexiblen flächigen Auflagestücks in einer Draufsicht und mehreren Schnittdarstellungen,
- Figur 3: eine dritte Ausführungsform eines flexiblen flächigen Auflagestücks in einer Draufsicht und einer Schnittdarstellung,
- Figur 4: eine vierte Ausführungsform eines flexiblen flächigen Auflagestücks in einer Draufsicht und einer Schnittdarstellung,
- Figur 5: eine fünfte Ausführungsform eines flexiblen flächigen Auflagestücks in einer Draufsicht und einer Schnittdarstellung,
- Figur 6: eine sechste Ausführungsform eines flexiblen flächigen Auflagestücks in einer Draufsicht und einer Schnittdarstellung,
- Figur 7: eine siebte Ausführungsform eines flexiblen flächigen Auflagestücks in einer Draufsicht und einer Schnittdarstellung.

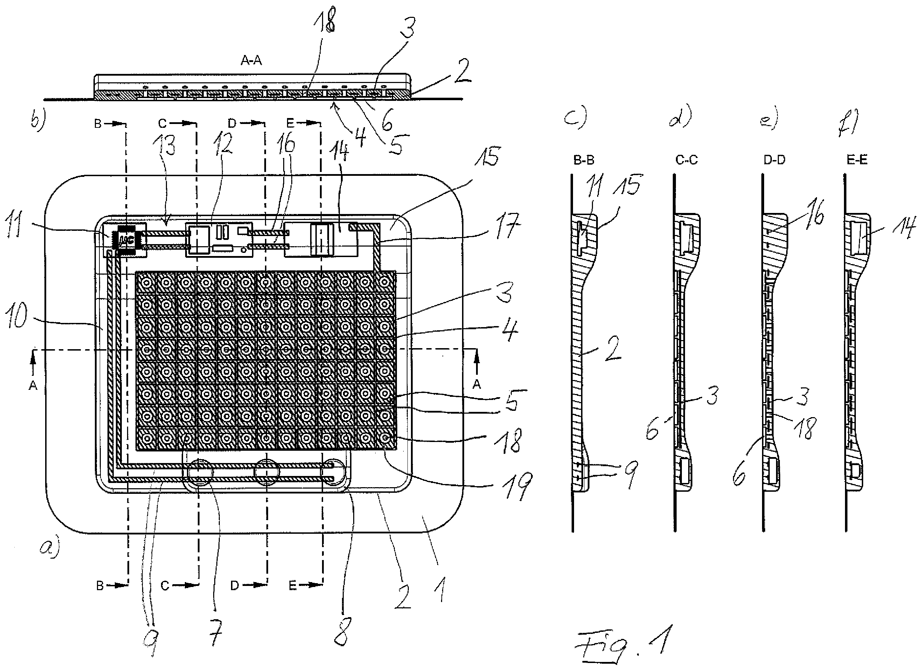

Das in Figur 1 dargestellte Ausführungsbeispiel ist in Figur 1a) in einer Ansicht von unten dargestellt, wobei im Gebrauch nicht sichtbare Teile im Innern des Auflagestücks dargestellt sind. Ferner ist das Ausführungsbeispiel mithilfe eines Längsschnitts A-A in Figur 1a) sowie mehrerer Querschnitte B-B (Figur 1c)), C-C (Figur 1d)), D-D (Figur 1e)) und E-E (Figur 1f)) dargestellt.

Das dargestellte Auflagestück weist eine im Wesentlichen rechteckige Grundform auf, in der ein Rand 1 umläuft. Der Rand 1 kann an seiner Unterseite haftklebend ausgebildet sein, um das Auflagestück beispielsweise auf der Haut eines Körperteils klebend befestigen zu können. Der umlaufende Rand 1 kann einstückig mit einer Dielektrikumsschicht 2 verbunden sein, die mit einer größeren Dicke als der umlaufende Rand 1 ausgebildet ist. In die Dielektrikumsschicht 2 ist eine Schicht aus leitendem Material als Elektrode 3 eingebettet, das heißt allseitig von dem Material der Dielektrikumsschicht 2 umgeben. Die Elektrode 3 weist in dem dargestellten Ausführungsbeispiel ebenfalls eine rechteckige Form auf, die sich jedoch allseitig nicht soweit erstreckt wie die Dielektrikumsschicht 2, sodass die Dielektrikumsschicht 2 mit Randabschnitten über die Elektrode 3 auf jeder Seite herausragt. Es ist klar, dass die dargestellte Grundform des Auflagestücks auch anders gestaltet sein kann, beispielsweise mehreckig, rund, oval o. ä. Im Bereich der Elektrode 3 ist die Dielektrikumsschicht 2 auf ihrer Unterseite in einer Gitterstruktur 4 ausgebildet, die aus schmalen, sich kreuzenden Stegen 5 besteht, wodurch im Querschnitt etwa quadratische, nach unten offene Kammern 6 ausgebildet werden. Die Stege 5 bilden trotz ihrer geringen Wandstärke eine stabile Gitterstruktur 4 aus, die als Abstandshalter dient, wenn das Auflagestück auf einer zu behandelnden Oberfläche aufliegt. Auf diese Weise kann sich im Gasraum (Luftraum) der Kammern 6 eine von der Elektrode 3 verursachte dielektrisch behinderte stabile Plasmaentladung ausbilden, mit der die Behandlung der Oberfläche erfolgt. Die durch die Gitterstruktur 4 vorhandene Strukturstabilität ermöglicht es, die Breite der Stege sehr klein zu halten, sodass der Luftraum in den Kammern 6 optimal groß ist. Die Breite der Stege beträgt beispielsweise weniger als 1/5 der senkrecht zu den Stegen gemessenen Ausdehnung der Kammern 6.

Es ist für den Fachmann ersichtlich, dass die Kammern 6, die in Figur 1 durch senkrecht zueinander verlaufende Stege 5 gebildet sind, auch andere Formen aufweisen können, beispielsweise Rautenformen, Sechseckformen (Wabenstruktur), usw. Damit der Vorteil der Stabilität der Gitterstruktur 4 erzielt wird, ist es zweckmäßig, in jeder Richtung der Dielektrikumsschicht 2 wenigstens vier, insbesondere wenigstens sechs und weiter insbesondere wenigstens acht Kammern 6 hintereinander angeordnet vorzusehen. Für den Fall, dass eine längliche Elektrodenanordnung aus Dielektrikumsschicht 2 und Elektrode 3 benötigt wird, ist es denkbar, in Breitenrichtung auch eine geringere Anzahl von Kammern 6 nebeneinander anzuordnen, wenn in Längsrichtung eine größere Anzahl von Kammern 6 vorgesehen wird. Die Anzahl der Kammern 6 auf der Unterseite der Dielektrikumsschicht 2 beträgt in üblichen Anwendungsfällen mindestens zwölf, insbesondere mindestens zwanzig und vielen Fällen mindestens vierzig. Das in Figur 1 dargestellte Ausführungsbeispiel weist in Längsrichtung dreizehn und in Breitenrichtung acht Kammern 6 auf, sodass sich eine Gesamtanzahl von hundertvier Kammern 6 ergibt.

In der in Figur 1 dargestellten Ausführungsform weist das Auflagestück keine Anschlüsse nach außen auf, ist also autark in der Lage, in den Kammern 6 ein Plasmafeld zu erzeugen, wenn die zu behandelnde Oberfläche, auf der das Auflagestück dann aufliegt, als Gegenelektrode fungiert. Das Auflagestück weist daher eine einzige Elektrode 3 auf, die mit einer Hochspannung versorgt werden muss, um ein Plasmafeld in den Kammern 6 zu erzeugen.

Für die Versorgung der Elektrode 3 sind in dem Auflagestück drei Batterien 7, hier in Form von Knopfzellen, vorgesehen. Die Batterien befinden sich in einem unteren Randstück 8 der Dielektrikumsschicht 2, das für die Aufnahme der Batterien wulstartig verdickt ausgebildet sein kann, wie dies in den Schnittdarstellungen C-C und D-D (Figuren 1d) und e)) erkennbar ist. Die Batterien 7 sind durch in die Dielektrikumsschicht 2 eingebettete Leiterbahnen 9 miteinander verbunden. Die Leiterbahnen 9 erstrecken sich über ein seitliches Randstück 10 der Dielektrikumsschicht bis zu einem Mikrocontrollerchip 11. Der Mikrocontrollerchip 11 bildet zusammen mit einem elektronischen Signalformer 12 ein Steuergerät 13. Der durch den Ausgang des Signalformers 12 gebildete Ausgang des Steuergeräts 13 ist mit dem Eingang einer Transformatorstufe 14 verbunden, die der Bildung einer Arbeits-Hochspannung von beispielsweise 15 kV aus einer Eingangsspannung von beispielsweise 250 V dient. Die Anordnung aus Steuergerät 13 und Transformatorstufe 14 befindet sich in einem oberen Randstück 15 der Dielektrikumsschicht 2.

Wie die Schnittdarstellungen der Figuren 1c) bis 1f) verdeutlichen, ist der obere Randabschnitt 15 zur Aufnahme der elektronischen Bauelemente ebenfalls verdickt gegenüber der Dielektrikumsschicht 2 im Bereich der Elektrode 3 ausgebildet. In die Randstücke 8, 10 und 15 erstreckt sich die Elektrode 3 nicht.

Der Mikrocontrollerchip 11, der Signalformer 12 und die Transformatorstufe 14 sind durch in die Dielektrikumsschicht 2 eingebettete Leiterbahnen 16 miteinander verbunden, die in gleicher Weise wie die Leiterbahnen 9 ausgebildet sind.

Die Verbindung des Ausgangs der Transformatorstufe 14 mit der Elektrode 3 erfolgt über eine für die Übertragung einer Hochspannung geeignete Hochspannungsleiterbahn 17, die als einstückiger Ansatz der Elektrode 3 ausgebildet sein kann.

Der Mikrocontrollerchip 11 erhält seine Versorgungsspannung aus den Batterien 7, die elektrisch in Serie geschaltet sein können, um die miteinander addierten Zellenspannungen als Versorgungsspannung des Mikrocontrollerchips 11 zur Verfügung zu stellen. Der Mikrocontrollerchip 11 steuert die Ausbildung von Wechselspannungspulsen im Signalformer 12, die aus der Versorgungspannung der Batterien von einigen V auf eine Wechselspannung von ca. 250 V Spitzenspannung hochgesetzt werden. Diese Wechselspannung gelangt auf die Transformatorstufe 14 zur Bildung von Hochspannungsimpulsen, beispielsweise mittels (nicht dargestellter) Zündstrecken, wobei sich die Hochspannungsimpulse (mit abwechselnder Polarität) aufgrund eines gewissen Schwingkreisverhaltens als Wechselspannungszüge mit stark abfallender Amplitude darstellen können. Durch die Hochspannungsimpulse wird die Elektrode 3 gegenüber der als Gegenelektrode wirkenden zu behandelnden Oberfläche abwechselnd auf ein hohes positives und negatives Potential gebracht, wodurch in dem in den Kammern 6 befindlichen Gas (insbesondere Luft) die gewünschte dielektrisch behinderte Plasmaentladung stattfinden kann.

Figur 1 lässt noch erkennen, dass die die Kammern 6 nach oben begrenzende Dielektrikumsschicht 2 mit Durchgangslöchern 18 versehen ist, durch die beispielsweise Fluid von der Oberfläche vor, während oder nach der Plasmabehandlung abgesaugt werden kann oder alternativ vor oder während der Behandlung ein Behandlungsgas in die Kammern 6 geleitet werden kann.

Um im Bereich der Durchgangsöffnung die Elektrode 3 mit der Dielektrikumsschicht 2 abzuschirmen, weist die Elektrode 3 zu jedem Durchgangsloch 18 eine Ausnehmung 19 auf, die größer ist als die Durchgangsöffnung 18, sodass die Wandung der Durchgangsöffnung 18 ununterbrochen von der dem Material der Dielektrikumsschicht 2 gebildet wird.

Auch wenn in dem dargestellten Ausführungsbeispiel jede Kammer mit einer Durchgangsöffnung 18 versehen ist, bedeutet dies nicht, dass eine derartige Ausgestaltung notwendig ist. Eine Absaugung von Fluid kann auch über eine deutlich geringere Anzahl von Durchgangsöffnungen 18 erfolgen. Dies gilt insbesondere dann, wenn die Stege 5 der Gitterstruktur 4 eine Fluidkommunikation zwischen den Kammern 6 - zumindest teilweise - ermöglichen. In dem dargestellten Ausführungsbeispiel ist jede Kammer 6 mit einer Durchgangsöffnung 18 versehen. Dies ermöglicht eine Ausbildung von Stegen 5 einer konstanten Höhe, sodass die Stege 5 bei der Auflage des Auflagestücks auf der zu behandelnden Oberfläche weitgehend abgeschlossene Kammern 6 bilden. Bei ungleichmäßig ausgebildeten Oberflächen ergibt sich dies auch dadurch, dass sowohl das Material der Dielektrikumsschicht 2 als auch das Material der Elektrode 3 flexibel sind, sodass sich das Auflagestück einer unregelmäßigen Oberfläche, wie beispielsweise einer Haut- oder Wundoberfläche, anpassen kann.

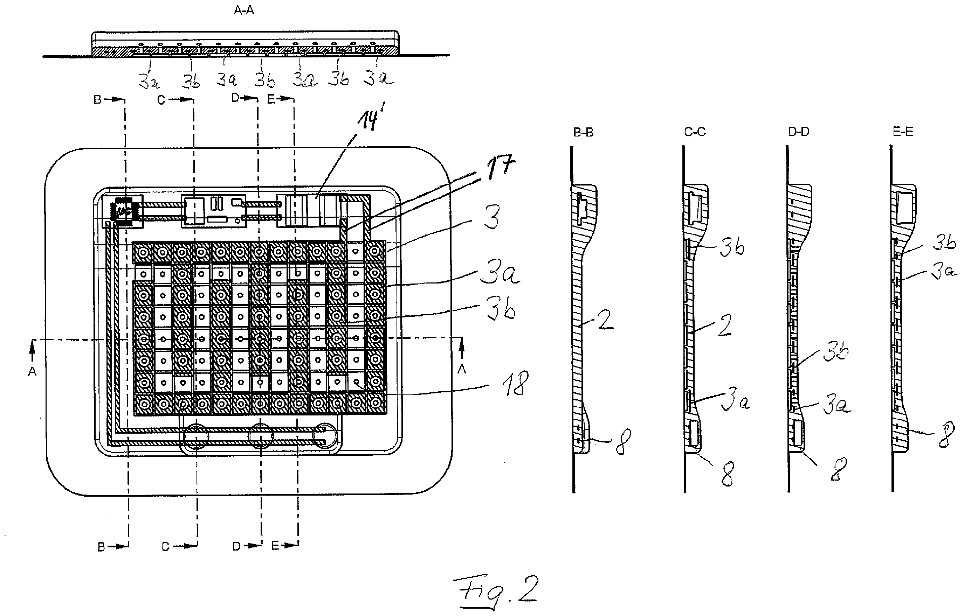

Die in Figur 2 dargestellte zweite Ausführungsform unterscheidet sich von der Ausführungsform der Figur 1 nur dadurch, dass die Elektrode 3 durch zwei Teilelektroden 3a, 3b gebildet ist, die kammförmig ineinandergreifend ausgebildet sind. Zwischen den Teilelektroden 3a, 3b befindet sich ein mäanderförmig ausgebildeter isolierender Streifen durch das Material der Dielektrikumsschicht, weil sich in diesem Bereich keine elektrisch leitende Elektrodenschicht befindet. Figur 2 verdeutlicht, dass diese Ausbildung der Elektrode 3 den übrigen Aufbau des Auflagestücks nicht ändert. Insbesondere können die Kammern 6 sowohl im Bereich der Teilelektroden 3a, 3b als auch im Bereich des isolierenden Streifens vorhanden sein. Ebenso sind die Durchgangslöcher 18 auch hier für jede Kammer 6 vorgesehen.

Die Teilelektrode 3a und 3b werden von der Transformatorstufe 14' mit zueinander umgepolten Hochspannungsimpulsen möglichst phasengleich versorgt. Dadurch entsteht ein Plasmafeld zwischen den Teilelektroden 3a, 3b gegenüber der durch die Oberfläche gebildeten Gegenelektroden, aber auch eine doppelt so große Spannungsdifferenz zwischen den beiden Teilelektroden 3a, 3b, wodurch die Plasmaausbildung durch das zwischen den Teilelektroden 3a und 3b vorhandene elektrische Feld noch verbessert wird.

Die Transformatorstufe 14' ist in diesem Fall mit zwei Transformatorspulen versehen, die zueinander umgekehrt gepolt sind und so jeweils eine der beiden Teilelektroden 3a, 3b mit den Spannungsimpulsen versorgen. Entsprechend befinden sich zwischen der Transformatorstufe 14' und den Teilelektroden 3a und 3b auch jeweils eine Hochspannungsleiterbahn 17.

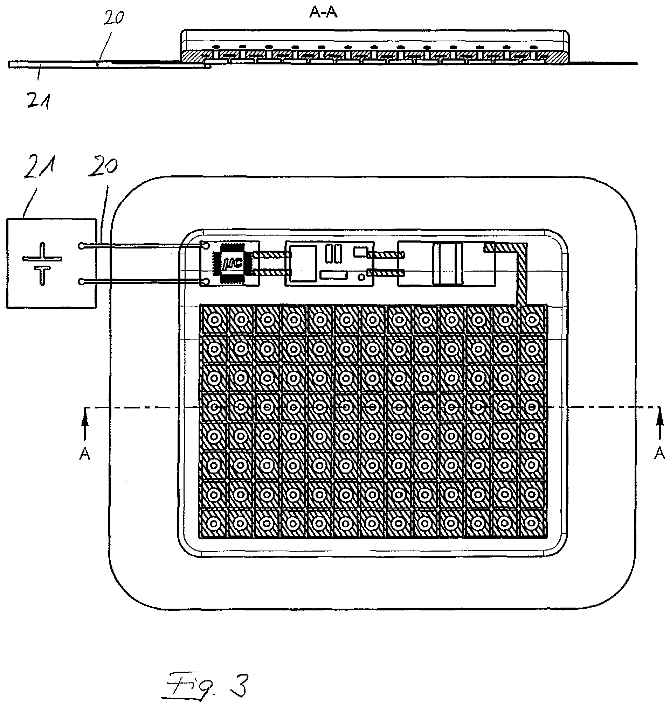

Das in Figur 3 dargestellte Ausführungsbeispiel entspricht dem Ausführungsbeispiel gemäß Figur 1 mit dem Unterschied, dass keine eigenen Batterien 7 vorgesehen sind. Vielmehr ist in diesem Ausführungsbeispiel das Auflagestück mit nach außen führenden Anschlüssen 20 versehen, an die eine Gleichspannungsquelle 21 anschließbar ist. Die Anschlüsse 20 können sich dabei an einem Ansatz des Auflagestücks befinden und entsprechend kontaktiert werden oder auch durch ein Anschlusskabel gebildet sein, mit dem die Verbindung zur Gleichspannungsquelle 21 hergestellt wird. Die Gleichspannungsquelle 21 ersetzt lediglich die Batterien 7, sodass der Aufbau und die Funktion des Auflagestücks unverändert bleibt. Da keine Batterien 7 in dem Auflagestück enthalten sein müssen, kann das untere Randstück 8 aus dem Ausführungsbeispiel gemäß Figur 1 entfallen.

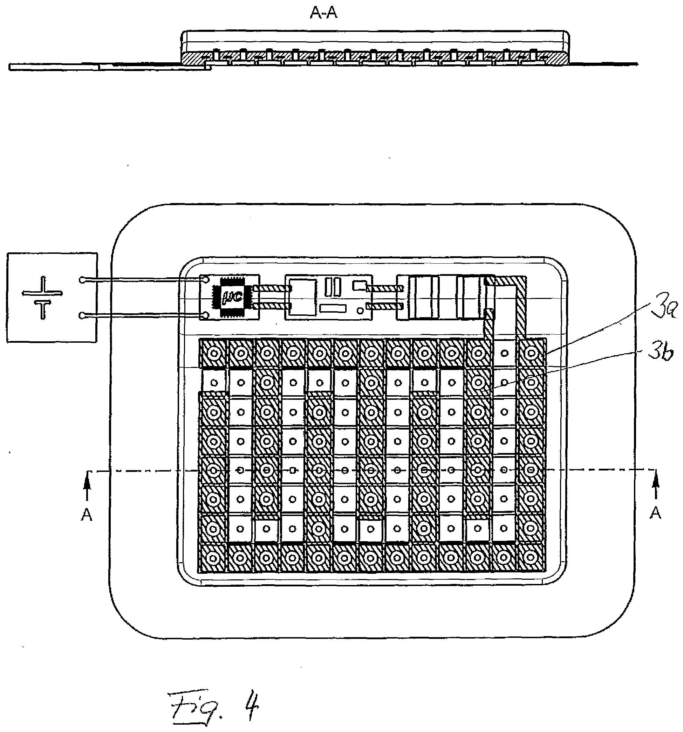

Das in Figur 4 dargestellte Ausführungsbeispiel ist mit dem Ausführungsbeispiel gemäß 3 identisch, bezieht sich jedoch auf ein Auflagestück mit zwei Teilelektroden 3a, 3b, während das Ausführungsbeispiel gemäß Figur 3 eine einzige Elektrode 3 betrifft. Auch diesbezüglich sind die Funktionen die gleichen wie anhand der Figuren 1 und 2 beschrieben.

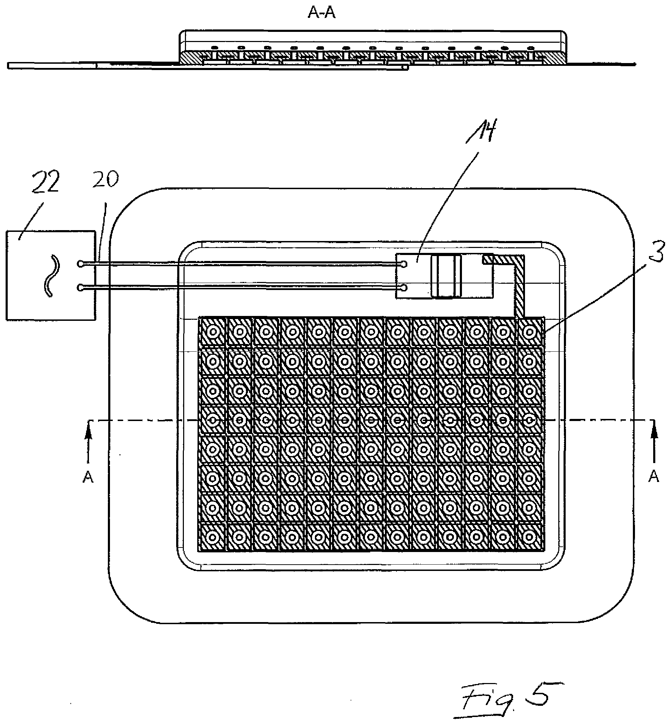

Das fünfte Ausführungsbeispiel gemäß Figur 5 enthält auf dem Ansatzstück nur noch die Transformatorstufe 14. Auch in dieser Ausführungsform enthält das Ansatzstück Anschlüsse 20 zum Anschluss eines externen Spannungsversorgungsgeräts, das hier durch eine Wechselspannungsversorgung 22 gebildet wird, aus der die Transformatorstufe 14 die geeigneten Hochspannungsimpulse für die Entstehung eines Plasmas zwischen der Elektrode 3 und der zu behandelnden Oberfläche generiert.

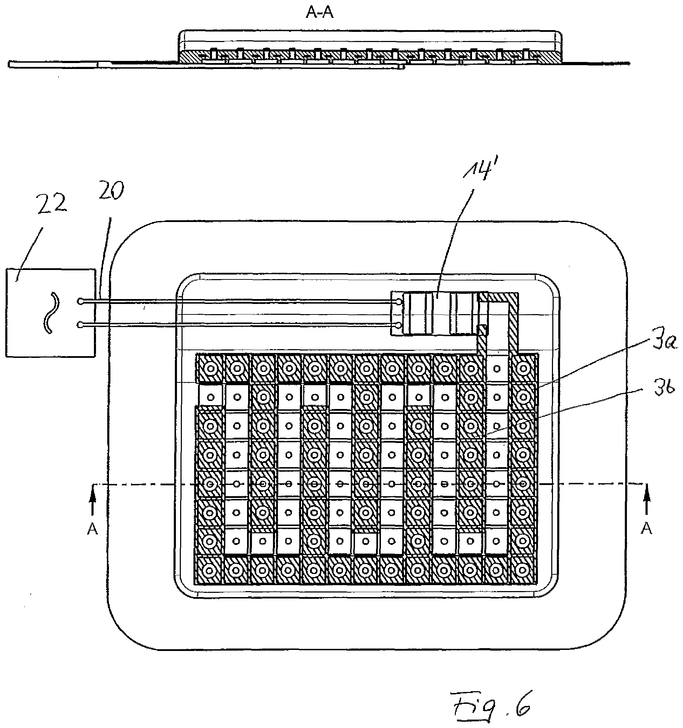

Gemäß Figur 6 ist der Anschluss einer Wechselspannungsversorgung unmittelbar an eine Transformatorstufe 14 auch für ein Ansatzstück mit zwei Teilelektroden 3a, ab - wie oben beschrieben - verwendbar. Bei den Ausführungsformen gemäß den Figuren 5 und 6 werden die Wechselspannungsversorgung und die Signalformung extern durchgeführt. Dennoch verbleibt der Vorteil, dass keine sicherheitstechnisch kritischen Hochspannungssignale auf das Ansatzstück übertragen werden müssen, da die Hochspannungssignale erst in der Transformatorstufe 14 innerhalb des Ansatzstücks erzeugt werden und auf kurzem Wege, beispielsweise mit den eingebetteten Hochspannungsleiterbahnen 17 auf die Elektrode 3 bzw. die Teilelektroden 3a, 3b geleitet werden. Wie beschrieben, können die Hochspannungsleiterbahnen 17 in die Dielektrikumsschicht eingebettet sein, sodass mit der Abschirmung der Elektrode 3 bzw. Teilelektroden 3a, 3b in gleicher Technologie auch die Isolierung der Hochspannungsleitungen 17 innerhalb der Dielektrikumsschicht 2 erfolgt.

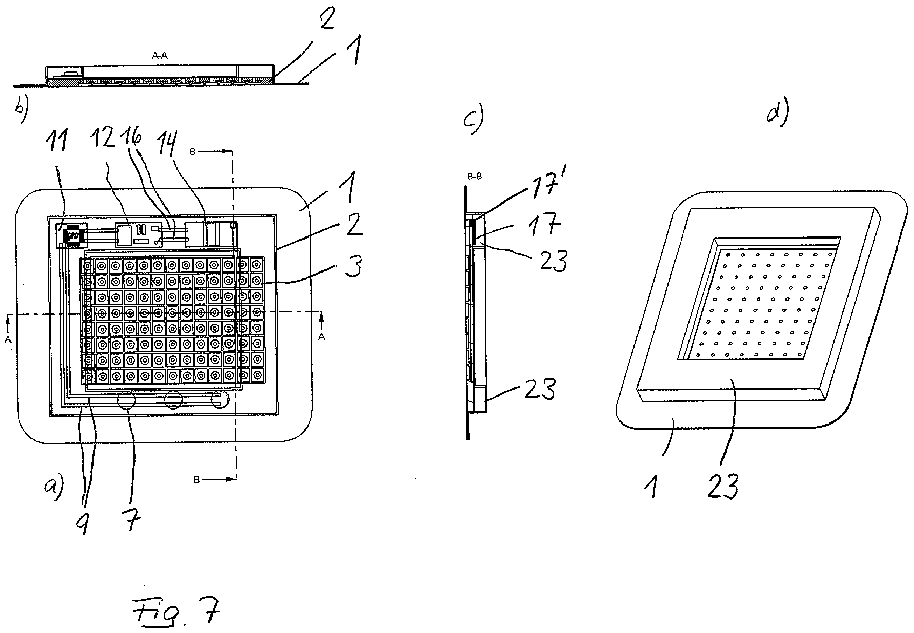

Das in Figur 7 dargestellte siebte Ausführungsbeispiel entspricht dem ersten Ausführungsbeispiel gemäß Figur 1, allerdings sind hier die Batterien 7, die Leiterbahnen 9, 16, der Mikrocontrollerchip 11, der Signalformer 12 und die Transformatorstufe 14 nicht von dem Material der Dielektrikumsschicht 2 umschlossen, sondern auf dem Material der Dielektrikumsschicht 2 montiert, wie dies insbesondere Figur 7b verdeutlicht. Die Leiterbahnen 9, 16 können dabei auf die Dielektrikumsschicht 2 direkt aufgebracht sein oder auf eine Folie gedruckt sein, die ihrerseits auf die Dielektrikumsschicht 2 aufgeklebt ist. Der elektrische Teil ist durch ein auf die Dielektrikumsschicht aufgebrachtes Gehäuse 23 abgedeckt, das einen streifenförmig am Rand der Dielektrikumsschicht 2 umlaufenden, unten offenen Kanal bildet, der durch die Dielektrikumsschicht 2 nach unten abgeschlossen ist. Das Gehäuse 23 besteht aus einem isolierenden Material und kann zur Erhaltung der für die Anpassung des Auflagestücks an unregelmäßige Oberflächen benötigte Flexibilität aus einem isolierenden, formbeständigen, jedoch weichelastischen Material, bspw. einem Elastomer, bestehen.

Die Bildung eines ringförmig geschlossenen Gehäuses 23 führt zu einer beliebigen Verwendbarkeit des Auflagestücks, ohne eine Vorzugsrichtung auszubilden. Es ist jedoch auch möglich, das Gehäuse nur an einem Rand streifenförmig auszubilden oder - je nach Bedarf - in L- oder U-Form zu gestalten.

Figur 7c) verdeutlicht, dass die Zuführung der Hochspannung zur Elektrode 3 mit einer Hochspannungsleiterbahn 17 erfolgt, die oberhalb der Dielektrikumsschicht 2 verläuft und durch eine Öffnung der Dielektrikumsschicht 2 einen in der Dielektrikumsschicht 2 geführten Ansatz der Elektrode 3 mit einem Vorsprung 17' kontaktiert. Auch hier wird die Hochspannung nur über eine kurze Strecke geführt und ist durch das Gehäuse 23 gegen Berührung und Überschlag unproblematisch schützbar.

Die Dielektrikumsschicht 2 ist in allen Ausführungsbeispielen bevorzugt dadurch herstellbar, dass zunächst eine untere Lage der Dielektrikumsschicht 2 gegossen wird, auf die die Elektrode 3 aufgelegt wird, woraufhin dann eine obere Lage der Dielektrikumsschicht aufgegossen wird, die sich mit der unteren Lage einstückig verbindet. Alternativ hierzu kann eine untere Lage der Dielektrikumsschicht vorgefertigt werden, dann die Elektrode 3 aufgelegt und anschließend eine obere Lage der Dielektrikumsschicht 2 in vorgefertigter Form aufgebracht werden. Die beiden Schichten können dann isolierend miteinander verklebt oder vorzugsweise spiegelverschweißt werden. In einer noch anderen Ausführungsform lässt sich die Dielektrikumsschicht 2 in einem Schritt einstückig durch Spritzgießen herstellen, wobei die Elektrode 3 in die Spritzgussform eingelegt wird.

In ähnlicher Weise können auch die elektrischen Bauelemente, wie Batterien 7, Mikrocontrollerchip 11, Signalformer 12 und Transformatorstufe 14 mit den Leiterbahnen 9, 16 und 17 in die Dielektrikumsschicht integriert werden. Die Verdickung der Dielektrikumsschicht 2 im unteren Randstück 8 und im oberen Randstück 15 kann beispielsweise bei der Fertigstellung der oberen Lage der Dielektrikumsschicht 2 im Spritzgussverfahren realisiert werden.

Das erfindungsgemäße Auflagestück kann in allen dargestellten Ausführungsformen als Einmalartikel ausgebildet und verwendet werden. In den Ausführungsformen der Figuren 1, 2 und 7 wird die gesamt Anordnung entsorgt, in den übrigen Ausführungsformen wird lediglich der Anschluss an ein externes Gerät gelöst. Im Falle des Anschlusses des Auflagestücks an eine Unterdruckquelle zum Zwecke der Absaugung von Wundsekret kann auf die Oberseite der Dielektrikumsschicht ein die abgesaugte Flüssigkeit absorbierendes Material angeordnet sein, beispielsweise unter einer das Absaugen unterstützenden luftdichten Folie.

Das erfindungsgemäße Auflagestück eignet sich insbesondere als Wundauflage, die für die gesamte Heildauer der Wunde auf der Wunde verbleiben kann, weil mit dem Mikrocontrollerchip 11 periodisch die dielektrisch behinderte Plasmabehandlung für eine benötigte Behandlungszeit ausgelöst werden kann, wodurch der gesamte Wundbereich immer wieder keimfrei gemacht werden kann, sodass eine beschleunigte Heilung der Wunde erreicht wird. Hierzu trägt auch eine durch die Plasmaentladung entstehende kontinuierliche Erhöhung der Mikrozirkulation im und um den Wundbereich und/oder in und um die intakte Haut bei.

## Patentansprüche

1. Flächiges flexibles Auflagestück mit einer mit einer Hochspannung versorgbaren Elektrodenanordnung für eine dielektrisch behinderte Plasmabehandlung einer zu behandelnden Oberfläche, wobei die Elektrodenanordnung wenigstens eine flächige Elektrode (3) und eine Auflagefläche für die zu behandelnde Oberfläche aufweisende Dielektrikumsschicht (2) aus einem flächigen flexiblen Material aufweist, die die wenigstens eine Elektrode (3) elektrisch von der zu behandelnden Oberfläche so abschirmt, dass nur ein dielektrisch behinderter Stromfluss zwischen der wenigstens einen Elektrode (3) und der zu behandelnden Oberfläche möglich ist, wenn in einem Gasraum zwischen der Elektrodenanordnung und der zu behandelnden Oberfläche ein Plasmafeld durch eine Hochspannung der Elektrode (3) entsteht, **dadurch gekennzeichnet, dass** das Auflagestück eine Hochspannungsstufe (14) zur Erzeugung der Hochspannung aufweist, deren Ausgang mit der wenigstens einen Elektrode (3) durch ein als leitender Abschnitt innerhalb der Dielektrikumsschicht (2) ausgebildetes Verbindungsstück (17, 17') auf dem Auflagestück verbunden ist.

2. Auflagestück nach Anspruch 1, **dadurch gekennzeichnet, dass** das Verbindungsstück eine Hochspannungsleiterbahn (17, 17') innerhalb der Dielektrikumsschicht (2) ist.

3. Auflagestück nach Anspruch 2, **dadurch gekennzeichnet, dass** die Hochspannungsleiterbahn (17, 17') aus einem spritzgegossenen Kunststoffmaterial mit leitenden Zusätzen besteht.

4. Auflagestück nach Anspruch 2, **dadurch gekennzeichnet, dass** die Hochspannungsleiterbahn (17, 17') eine in die Dielektrikumsschicht (2) eingebrachte metallische Leiterbahn ist.

5. Auflagestück nach einem der Ansprüche 1 bis 4, **gekennzeichnet durch** Anschlüsse (20) für eine Wechsel-Versorgungsspannung, die auf die Hochspannungsstufe (14) gelangt.

6. Auflagestück nach einem der Ansprüche 1 bis 4, **dadurch gekennzeichnet, dass** es Batterien (7) für eine Gleichspannungsversorgung und eine Steuerschaltung (13) zur Umwandlung der Gleichspannung in Wechselspannungssignale einer höheren Spitzenspannung aufweist, die auf die Hochspannungsstufe (14) gelangen.

7. Auflage nach einem der Ansprüche 1 bis 6, **dadurch gekennzeichnet, dass** die Elektrodenanordnung wenigstens zwei Teilelektroden (3a, 3b) aufweist, die phasenverschoben mit der Wechsel-Hochspannung versorgbar sind.

8. Auflagestück nach einem der Ansprüche 1 bis 7, **dadurch gekennzeichnet, dass** es als eine Wundauflage mit einem wundverträglichen Material gebildet ist.

9. Auflagestück nach Anspruch 8, **dadurch gekennzeichnet, dass** auf die Auflagefläche des Dielektrikums ein wundverträgliches Material aufgebracht ist.

10. Auflagestück nach einem der Ansprüche 1 bis 9, **dadurch gekennzeichnet, dass** die Dielektrikumsschicht (2) als Spritzgussteil ausgebildet ist und sowohl die wenigstens eine Elektrode (3) als auch das Hochspannungsteil (14) allseitig umschließt.

## Claims

1. Planar flexible application piece having an electrode arrangement, which can be supplied with a high voltage, for a dielectric barrier plasma treatment of a surface to be treated, the electrode arrangement comprising at least one planar electrode (3) and a dielectric layer (2) of a planar flexible material having a bearing face for the surface to be treated, said dielectric layer electrically shielding the at least one electrode (3) from the surface to be treated in such a way that only a dielectric barrier current can flow between the at least one electrode (3) and the surface to be treated when a plasma field is formed in a gas space between the electrode arrangement and the surface to be treated by a high voltage of the electrode (3), **characterized in that** the application piece comprises a high-voltage stage (14) for generating the high voltage, the output of which is connected to the at least one electrode (3) by a connecting piece (17, 17'), which is configured as a conductive section inside the dielectric layer (2), on the application piece.

2. Application piece according to Claim 1, **characterized in that** the connecting piece is a high-voltage conductor track (17, 17') inside the dielectric layer (2) .

3. Application piece according to Claim 2, **characterized in that** the high-voltage conductor track (17, 17') consists of an injection-molded plastic material having conductive additives.

4. Application piece according to Claim 2, **characterized in that** the high-voltage conductor track (17, 17') is a metallic conductor track introduced into the dielectric layer (2).

5. Application piece according to one of Claims 1 to 4, **characterized by** terminals (20) for an AC voltage supply which passes to the high-voltage stage (14).

6. Application piece according to one of Claims 1 to 4, **characterized in that** it comprises batteries (7) for a DC voltage supply and a control circuit (13) for converting the DC voltage into AC voltage signals of a higher peak voltage, which signals pass to the high-voltage stage (14).

7. Application piece according to one of Claims 1 to 6, **characterized in that** the electrode arrangement comprises at least two partial electrodes (3a, 3b), which can be supplied in a phase-shifted manner with the AC high voltage.

8. Application piece according to one of Claims 1 to 7, **characterized in that** it is formed as a wound dressing comprising a wound-compatible material.

9. Application piece according to Claim 8, **characterized in that** a wound-compatible material is applied onto the application face of the dielectric.

10. Application piece according to one of Claims 1 to 9, **characterized in that** the dielectric layer (2) is configured as an injection-molded part and encloses both the at least one electrode (3) and the high-voltage part (14) on all sides.

## Revendications

1. Élément d'appui plat flexible comportant un ensemble d'électrodes pouvant être alimenté en haute tension pour un traitement par plasma à barrière diélectrique d'une surface à traiter, l'ensemble d'électrodes comprenant au moins une électrode plate (3) et une couche diélectrique (2) en un matériau plat flexible qui présente une surface d'appui pour la surface à traiter et qui protège électriquement ladite au moins une électrode (3) vis-à-vis de la surface à traiter de manière à permettre uniquement un flux de courant à barrière diélectrique entre ladite au moins une électrode (3) et la surface à traiter lorsqu'un champ de plasma est généré dans un espace gazeux entre l'ensemble d'électrodes et la surface à traiter par une haute tension de l'électrode (3),
**caractérisé en ce que**
l'élément d'appui présente un étage haute tension (14) pour générer la haute tension, dont la sortie est reliée à ladite au moins une électrode (3) par une pièce de liaison (17, 17') disposée sur l'élément d'appui et réalisée sous forme de portion conductrice à l'intérieur de la couche diélectrique (2).

2. Élément d'appui selon la revendication 1,
**caractérisé en ce que**
la pièce de liaison est une piste conductrice haute tension (17, 17') à l'intérieur de la couche diélectrique (2).

3. Élément d'appui selon la revendication 2,
**caractérisé en ce que**
la piste conductrice haute tension (17, 17') est constituée d'une matière plastique moulée par injection ayant des additifs conducteurs.

4. Élément d'appui selon la revendication 2,
**caractérisé en ce que**
la piste conductrice haute tension (17, 17') est une piste conductrice métallique insérée dans la couche diélectrique (2).

5. Élément d'appui selon l'une des revendications 1 à 4,
**caractérisé par** des connexions (20) pour une tension d'alimentation alternative qui atteint l'étage haute tension (14).

6. Élément d'appui selon l'une des revendications 1 à 4,
**caractérisé en ce que**
il comprend des batteries (7) pour une tension d'alimentation continue et un circuit de commande (13) pour convertir la tension continue en signaux de tension alternative d'une tension de crête plus élevée, qui atteignent l'étage haute tension (14).

7. Élément d'appui selon l'une des revendications 1 à 6,
**caractérisé en ce que**
l'ensemble d'électrodes comprend au moins deux électrodes partielles (3a, 3b) qui peuvent être alimentées par la haute tension alternative en déphasage.

8. Élément d'appui selon l'une des revendications 1 à 7,
**caractérisé en ce que**
il est réalisé sous forme de pansement pour plaies avec un matériau compatible avec la plaie.

9. Élément d'appui selon la revendication 8,
**caractérisé en ce que**
un matériau compatible avec la plaie est appliqué sur la surface d'appui du diélectrique.

10. Élément d'appui selon l'une des revendications 1 à 9,
**caractérisé en ce que**
la couche diélectrique (2) est réalisée sous forme de pièce moulée par injection et entoure de tous côtés aussi bien ladite au moins une électrode (3) que la partie haute tension (14).
